Europäisches Patentamt

European Patent Office

Office européen des brevets

(11) Veröffentlichungsnummer : **0 457 789 B1**

(12) **EUROPÄISCHE PATENTSCHRIFT**

(45) Veröffentlichungstag der Patentschrift :
**11.11.92 Patentblatt 92/46**

(51) Int. Cl.$^5$ : **C12Q 1/18, C12Q 1/04**

(21) Anmeldenummer : **90902620.5**

(22) Anmeldetag : **08.02.90**

(86) Internationale Anmeldenummer :
**PCT/DE90/00083**

(87) Internationale Veröffentlichungsnummer :
**WO 90/09454 23.08.90 Gazette 90/20**

(54) **VERFAHREN ZUR SCHNELLEN PRÜFUNG DER WIRKSAMKEIT VON AGENZIEN AUF MIKROORGANISMEN.**

(30) Priorität : **09.02.89 DE 3903778**

(43) Veröffentlichungstag der Anmeldung :
**27.11.91 Patentblatt 91/48**

(45) Bekanntmachung des Hinweises auf die
Patenterteilung :
**11.11.92 Patentblatt 92/46**

(84) Benannte Vertragsstaaten :
**CH DE GB LI**

(56) Entgegenhaltungen :
**EP-A- 0 139 143**
**EP-A- 0 151 855**
**Journal of bacteriology, Band 65, 1953 (Baltimore, US) J.H. Bailey et al "Effect of certain antibacterial agents on theinfrared spectrum of micrococcus pyogenes var. aureus", page 750**

(56) Entgegenhaltungen :
**Infrared Physics, Band24, n 2/3, 1984 (Oxford, GB) D. Naumann "Some ultrastructural information on intact, living bacterialcells and related cell-wall fragments as given by FTIR", pages 233-238**

(73) Patentinhaber : **Bruker Analytische Messtechnik GmbH**
**Silberstreifen**
**W-7512 Rheinstetten 4 (DE)**

(72) Erfinder : **NAUMANN, Dieter**
**Mariannenplatz 22**
**W-1000 Berlin 36 (DE)**
Erfinder : **LABISCHINSKI, Harald**
**Koenigsallee 43 a**
**W-1000 Berlin 33 (DE)**

(74) Vertreter : **Patentanwälte Kohler - Schmid + Partner**
**Ruppmannstrasse 27**
**W-7000 Stuttgart 80 (DE)**

EP 0 457 789 B1

## Beschreibung

Die Erfindung betrifft ein Verfahren zur schnellen Prüfung der Wirksamkeit von Agenzien auf Mikroorganismen, wie Bakterien und Pilzen.
Die Agenzien können Wachstumsstoffe sein. Sie sind insbesondere Hemmstoffe, wie Antibiotika und Chemotherapeutika, die zur Inhibierung des Wachstums und/oder der Abtötung von Mikroorganismen eingesetzt werden.

In der Chemotherapie steht eine wachsende Vielzahl von Chemotherapeutika und Antibiotika aus verschiedenen Präparatgruppen zur Verfügung, um Mikroorganismen, z.B. hinsichtlich ihrer Rolle als Krankheitserreger, zu bekämpfen. Trotz einer ständigen Weiterentwicklung der Produktpalette an Antibiotika und Chemotherapeutika (im weiteren auch als Hemmstoff oder Agenz bezeichnet) stellt die sich gleichfalls weiterentwickelnde Resistenz vieler Mikroorganismen gegen diese Hemmstoffe ein großes gesundheitspolitisches Problem dar. Es ist daher für die effektive Bekämpfung von Mikroorganismen durch Chemotherapeutika und/oder Antibiotika, z.B. im Falle der gezielten klinischen Bekämpfung von Infektionskrankheiten, unumgänglich, eine Prüfung der betreffenden Mikroorganismen nach Isolierung auf ihre Empfindlichkeit bzw. Resistenz gegen eine Vielzahl von Chemotherapeutika und/oder Antibiotika durchzuführen. Die dabei erhaltenen Aussagen über das Resistenzverhalten eines Mikroorganismus sind nicht nur für die klinische Therapie, sondern auch für epidemiologische Untersuchungen und für die medizinische Forschung allgemein von entscheidender Bedeutung. Derartige Prüfungen fallen daher in außerordentlich hoher Anzahl in der klinischen Routine, wie auch in der biologischen und medizinischen Forschung an. Bei der Durchführung der Empfindlichkeitstests bedient man sich in der Regel beim bisherigen Stand der Technik einer direkten, einfachen, optischen Verfolgung des Wachstums einer Bakterienkultur, jeweils mit und ohne Antibiotikum, indem z.B. die Trübung eines Kulturröhrchens (im sog. Reihenverdünnungstest) einer Kontrollprobe und weiterer Proben mit unterschiedlichen Dosen des verwendeten Chemotherapeutikums oder Antibiotikums nach einer Kulturdauer von etwa 20 Stunden durch Betrachtung bewertet wird. Weniger aufwendig sind die sog. Diffusionstests, bei denen ein sog. "Bakterienrasen" gleichmäßig auf eine Agarplatte aufgetragen wird, wobei die Agarplatte mit einem Papierfilter belegt wird, das mit einer standardisierten Menge des zu testenden Hemmstoffes getränkt ist. Nach ca. 24-stündiger Bebrütung kann aus dem Durchmesser des optisch erkennbaren Hemmhofes um die Filterscheibe auf die Empfindlichkeit des Mikroorganismus rückgeschlossen werden. Ohne sich bisher stark in der Praxis durchgesetzt zu haben, werden als weitere Alternative der Empfindlichkeitsprüfung automatisierte Systeme zur Verfolgung der optischen Dichte der Mikroorganismen-Kultur bei verschiedenen Antibiotikakonzentrationen im Vergleich zu einer unbehandelten Kontrollkultur eingesetzt, die im Einzelfall Aussagen nach etwa 5 bis 10 Stunden erlauben sollen.

Der dargestellte Stand der Technik (für eine Übersicht vgl. z.B. Antibiotics in Laboratory Medicine, Second Edition, V. Lorian, M.D., Herausgeber: Verlag Williams & Wilkins, Baltimore, London, Los Angeles, Sydney, 1986) bringt für den zielgerichteten Einsatz von Chemotherapeutika und Antibiotika vor allem aus zwei Gründen erhebliche Beschränkungen mit sich. Zum einen ist der Zeitbedarf für die Durchführung der Empfindlichkeitsprüfung noch immer so hoch, daß oftmals eine ungezielte Bekämpfung der Mikroorganismen mit z.B. Breitbandantibiotika notwendig ist, was einen entsprechenden Sektionsdruck in Richtung weiterer Resistenzen der Mikroorganismen, sowie ggf. sogar ein Therapieversagen nach sich ziehen kann; zum anderen sind die konventionellen optischen Methoden in vielen Fällen nicht aussagekräftig genug, da eine essentielle Schädigung der Mikroorganismen sich zum Teil kaum auf die optischen Eigenschaften einer Kultur auswirkt.

Die Infrarotspektroskopie ist in der Vergangenheit auch für die Untersuchung von Mikroorganismen hinsichtlich einer Identifizierung und Differenzierung eingesetzt worden (vgl. z.B. Norris, K.P., J.Hyg. 57, 326 (1959) und Giesbrecht, P., Naumann, D., Labischinski, H. in: Habermehl: Rapid Methods and Automation in Microbiology and Immunology, S. 198-206, Berlin: Springer Verlag (1985); Naumann, D., Fijala, V., Labischinski, H., Mikrochim. Acta [Wien] I, 373-377 (1988) und DE-A1-32 47 891). Bei der IR-Spektroskopie von derart komplexen Proben treten aber grundsätzliche sowie präparative und verfahrenstechnische Probleme auf.

Der Erfindung liegt die Aufgabe zugrunde, ein Verfahren zur Prüfung der Empfindlichkeit von Mikroorganismen gegenüber Agenzien der eingangs erwähnten Art, insbesondere gegenüber Chemotherapeutika und/oder Antibiotika zu entwickeln, durch das die Möglichkeit geschaffen wird, die unterschiedlichsten Mikroorganismen auf diese Empfindlichkeit mit sehr geringem Zeitaufwand sicher und präzise zu prüfen. Das erfindungsgemäße Verfahren soll auch in der Praxis der mikrobiologischen Laboratorien im Routinebetrieb effektiv einsetzbar sein.

Diese Aufgabe wird erfindungsgemäß durch die im Patentanspruch 1 angegebenen Maßnahmen gelöst.

Es wurde gefunden, daß bei Wirksamkeit eines Agenz schon nach kurzer Zeit während des Bebrütens bzw. Kultivierens eines Mikroorganismus charakteristische Veränderungen am Mikroorganismus selbst und/oder im Kulturmedium auftreten, die durch Infrarotspektren feststellbar sind. Die Zeitdauer, bis zu der diese Veränderungen in einer für die Auswertung der Spektren geeigneten Größe vorliegen, hängt von der Wirksamkeit des jeweiligen Agenz und von der Vermehrungsgeschwindigkeit des jeweiligen Mikroorganismus ab und kann durch Vorversuche ermittelt werden. In der Regel reichen für die Kultivierung Zeitdauern von weniger als 5 Stunden, insbesondere weniger als 4 Stunden aus, Normalerweise liegt die Zeitdauer zwischen 5 Minuten und 2 Stunden. Aus Sicherheitsgründen kann während einer Zeitdauer zwischen 30 Minuten und 1 Stunde kultiviert werden, obwohl ca. 30 Minuten in der Regel ausreichen. Ein wesentlicher Gesichtspunkt der Erfindung ist darin zu sehen, daß während der Einwirkung des Agenz auf den Mikroorganismus nicht nur quantitative Veränderungen, sondern insbesondere auch Veränderungen qualitativer Art stattfinden. Diese qualitativen Veränderungen am Mikroorganismus und/oder im Kulturmedium lassen sich durch die vergleichende IR-Spektroskopie leichter feststellen als quantitative Veränderungen und ermöglichen deshalb eine Wirksamkeitsbestimmung schon in einem sehr frühen Einwirkungsstadium des Agenz. Es braucht also nicht abgewartet zu werden, bis z.B. das weitere Wachstum des Mikroorganismus gehemmt ist (bakteriostatische Wirkung) oder bis der Mikroorganismus abgetötet ist (bakteriozide Wirkung). Vielmehr ist die Wirksamkeitsbestimmung bereits im Stadium einer ersten Veränderung des Mikroorganismus möglich. Hierzu können gezielt bestimmte charakteristische Veränderungen herangezogen werden, z.B. Abbau von Proteinen oder Zerfall von Zellwandbestandteilen, Auftreten von neuen Produkten, die durch Zerfall oder Synthese entstehen. Diese Produkte können in den Mikroorganismen und/oder im Kulturmedium auffindbar sein. Diese charakteristischen Veränderungen erlauben zusätzlich die Aufklärung oder Feststellung des Wirkungsmechanismus, was ebenfalls Gegenstand der Erfindung ist und worauf nachfolgend noch näher eingegangen wird. Ferner ist durch die Erfindung nicht nur die Feststellung möglich, ob ein Agenz wirksam ist, sondern auch wie stark es wirksam ist.

Der Vergleich der Spektren der in Gegenwart der Agenzien kultivierten Testproben erfolgt vorzugsweise mit unter gleichen Bedingungen und während der gleichen Zeitdauer kultivierten Kontrollproben, die durch Kultivieren des gleichen Mikroorganismus ohne Zusatz des Agenz erhalten werden. Dadurch lassen sich die Veränderungen am Mikroorganismus und-/oder im Kulturmedium am besten beurteilen. In besonderen Fällen ist auch ein Vergleich mit Spektren des Mikroorganismus und/oder des Kulturmediums, die vor oder bei Beginn der Bebrütung aufgenommen werden, möglich. Solche vor Beginn der Kultivierung aufgenommenen Spektren werden, wenn sie zum Vergleich herangezogen werden, vorzugsweise zusätzlich zum Vergleich mit dem Spektrum der Kontrollprobe herangezogen. Der Vergleich mit einem Spektrum, das vor Beginn bzw. zu Beginn der mit dem Agenz versetzten Prüfprobe gemacht wird, hat beispielsweise den Vorteil, daß Banden, die nicht durch die Wirkung des Agenz, sondern durch das Agenz selbst erzeugt werden, festgestellt und berücksichtigt werden können.

Vorteilhafte Weiterentwicklungen und Merkmale des erfindungsgemäßen Verfahrens ergeben sich aus den Unteransprüchen in Verbindung mit der nachfolgenden Beschreibung. Hierbei können die einzelnen Merkmale für sich allein oder in Kombination miteinander bei den einzelnen Ausführungen verwirklicht sein.

Geeignet verwendbar im Zusammenhang mit dem erfindungsgemäßen Verfahren sind IR-Geräte mit in den letzten Jahren erfolgten meßtechnischen Weiterentwicklungen der IR-Spektroskopie, insbesondere der Erhöhung ihrer Sensitivität, Wellenlängengenauigkeit, Schnelligkeit und Digitalisierbarkeit aller spektralen Daten und ihrer damit gegebenen Computer-Kompatibilität.

Die Empfindlichkeitsprüfung erfolgt daher besonders vorteilhaft auf einem Fourier-Transform-Infrarotspektrometer (FT-IR). Die IR-Geräte können zum Zweck der Spektrenspeicherung und des Spektrenvergleichs mit verschiedenen, an sich bekannten elektronischen Speichermedien, ggf. auch mit einem Rechneranschluß ausgerüstet sein. Eine besonders effektive Nutzung wird erreicht, wenn zur Strahlfokussierung "Beam-Kondensoren" und/oder IR-Mikroskope zur Verfügung stehen.

Von besonderer Bedeutung für das neue Verfahren ist, daß mit Hilfe der IR-Spektroskopie eine Art "chemische Totalanalyse" der Probe möglich wird, so daß nicht - wie beim bisherigen Stand der Technik - lediglich quantitative Einzelaspekte (wie z.B. Erniedrigung der optischen Dichte einer Bakterienkultur), sondern nach Wunsch erstmals auch qualitative Aspekte durch Nutzung der spektralen Zerlegung der Information und Analyse einzelner und/oder mehrerer kleiner, wohl definierter Spektralbereiche erhältlich sind. Aus dem gleichen Grund ist es mit Hilfe des erfindungsgemäßen Verfahrens auch prinzipiell möglich, die Empfindlichkeitsprüfung entweder durch spektralen Vergleich der Mikroorganismen mit und ohne Behandlung mit dem zu prüfenden Agenz oder aber auch durch spektralen Vergleich der bei der Prüfung verwendeten Kulturmedien selbst vorzunehmen, da bei Wirkung des Agenz, insbesondere Antibiotikums und/oder Chemotherapeutikums nicht nur die chemische Zusammensetzung der Mikroorganismen selbst, sondern auch die des verwendeten Kulturmediums sich von der entsprechenden Kontrollprobe (d.h. Wachstum ohne Hemmstoffzusatz) in IR-spektroskopisch zu erfassender, charakteristischer Weise verändern. Überraschenderweise erlaubt sogar die Vermessung von Aliquoten der gesamten Kultur, also Mischungen von Mikroorganismen mit den verwendeten Nährmedien, die Bestimmung der Empfindlichkeit, so daß in diesem Falle durch Verringerung der Präparationszeit (keine Trennung von Nährmedien und Mikroorganismen nötig) eine weitere vorteilhafte Zeiteinsparung erreicht werden kann. Besonders vorteilhaft ist dabei in allen diesen Fällen die Möglichkeit, nicht nur die Wirkung des zu prüfenden Agenz, insbesondere Hemmstoffs zu erfassen, sondern auch Aussagen über den Wirktyp (z.B. statischer oder lytischer Wirktyp) bzw. sogar über den Wirkmechanismus (z.B. Hemmung oder Stimulierung der Proteinsynthese u.ä.) zu erhalten.

Zur Durchführung des erfindungsgemäßen Verfahrens werden zunächst die zu untersuchenden, vorzugsweise in Reinkultur vorliegenden Mikroorganismen in ein an sich beliebiges, in der Mikrobiologie übliches Kulturmedium, wie z.B. eine flüssige Nährlösung oder auch z.B. auf ein festes Nährmedium überführt und in getrennten Ansätzen einmal ohne und einmal mit Zusatz des zu prüfenden Agenz, insbesondere Hemmstoffs kultiviert. Nach der gewünschten Kultivierungsdauer von z.B. 30 - 60 Minuten werden aus beiden Ansätzen die Mikroorganismen vom Kultivierungsmedium, z.B. durch Zentrifugation im Fall flüssiger Kulturmedien, oder aber auch z.B. durch Abnahme mit einer Platinöse im Fall fester Nährböden, getrennt. Alternativ kann, z.B. mit einer Mikroliterpipette, aber auch im Durchflußverfahren bei Verwendung einer der üblichen IR-Flüssigküvetten, die gesamte Kultur unter Einschluß von Medium und Mikroorganismen für die eigentliche Messung entnommen werden. Anschließend wird das Infrarotspektrum der Probe vorzugsweise auf einem FT-IR-Gerät registriert. Prinzipiell kann dabei jede der an sich bekannten IR-spektroskopischen Meßtechniken und alle an sich in der IR-Spektroskopie üblicherweise benutzten optischen Materialien eingesetzt werden. Besonders bewährt haben sich die Transmissions-, die innere und die äußere Reflexionsspektroskopie und optische Materialien, wie z.B. KRS 5, ZnSe, ZnS, AgCl polierte Stahlplatten, Alu- und Silberfolien. Wichtig bei der Festlegung der Parameter der Spektrengewinnung ist lediglich, daß für beide Ansätze, mit und ohne Agenz, identische Bedingungen gewählt werden. Die optischen Träger sind vorzugsweise so gefertigt, daß ausschließlich durch festgelegte Probenfenster das IR-Licht transmittiert bzw. reflektiert wird. Alle anderen Bereiche bleiben ausgeblendet. Die für die Aufnahme der IR-Spektren benötigten Substanzmengen und ihre flächenmäßige Ausdehnung können sehr klein gehalten werden. Je nach vorgegebenen Bedingungen (z.B. Spektroskopieren mit und ohne Strahlenkondensator bzw. IR-Mikroskop) können Substanzmengen zwischen $10^{-2}$ bis $10^{-7}$ mg Probesubstanz eingesetzt werden. Die Durchmesser der durchstrahlten Probenfelder variieren dementsprechend, z.B. zwischen 3 mm und 10 μm. Die Untergrenze entspricht einer Anzahl von ca.

100 Bakterien.

Die Beurteilung der Wirksamkeit des untersuchten Agenz auf den jeweiligen Mikroorganismus erfolgt dann durch Vergleich der Spektren aus beiden Ansätzen (d.h. Kultivierung ohne und mit Anwesenheit des Agenz). Dabei können, je nach Wunsch des Experimentators und Anforderung an das Prüfungsergebnis (z.B. bloße Aussage über Wirkungseintritt oder verfeinerte Aussagen über Wirktyp und/oder Wirkmechanismus, Zeitbedarf der Analyse bei zeitkritischen Prüfungen z.B. für einen schnellstmöglichen gerichteten Therapiebeginn im Fall der Bekämpfung von Infektionskrankheiten), unterschiedliche Vergleichstechniken entweder einzeln oder auch kombiniert zum Einsatz kommen. In der einfachsten Variante des Verfahrens kann die Beurteilung der Wirkung des verwendeten Agenz durch die Bestimmung des Verhältnisses bzw. der Differenz der Integrale der Absorptionen über den gesamten erfaßten Spektralbereich aus beiden Ansätzen (d.h. Proben nach Kultivierung mit und ohne Anwesenheit des Hemmstoffes) erfolgen. Erfahrungsgemäß ist ein verbessertes, d.h. vor allem auch bereits nach kürzerer Kultivierungsdauer erhältliches Resultat erreichbar, wenn die Integration nur auf einen und/oder mehrere kleinere Bereiche von insbesondere 100 - 600 Wellenzahlen beschränkt wird, wobei bei genügend klein gewählten Bereichen anstelle der Integration auch die Bestimmung des maximalen Absorptionswertes in dem oder den gewählten Wellenzahlbereich(en) genutzt werden kann. Durch Beschränkung des IR-Spektrums auf bestimmte charakteristische Wellenbereiche ist auch eine weitere Verkürzung der Verfahrensdauer möglich.

Die Auswahl des oder der geeigneten Wellenzahlbereiche kann z.B. durch visuelle Inspektion der Spektren (z.B. Auswahl der Bereiche mit den stärksten Veränderungen) oder aber z.B. auch in Abhängigkeit vom Wirkmechanismus erfolgen. So kann beispielsweise bei Testung eines Proteinbiosynthesehemmers, wie z.B. Chloramphenicol oder Tetrazyklin o.ä., zweckmäßigerweise ein Vergleich derjenigen Wellenzahlbereiche durchgeführt werden, in denen Absorptionsbanden, wie z.B. Amid I und II liegen, die auf die Menge des ingesamt gebildeten Proteins des untersuchten Mikroorganismus ansprechen. Im Falle einer Beeinflussung der Membranstruktur der Mikroorganismen, oder auch z.B. des Nukleinsäurestoffwechsels, können in analoger Weise bekannte Absorptionsbanden, die z.B. auf $CH_2$-Gruppen bzw. Phosphat ansprechen, genutzt werden. Besonders vorteilhaft - insbesondere auch bei nicht bekanntem Wirktyp des Hemmstoffes - ist eine vorhergehende Spektrenaufarbeitung durch z.B. Differenz- oder Derivativspektroskopie, Spektrenfilterung oder auch z.B. durch Spektrendekonvolution und andere Verfahren zur Erhöhung des spektralen Kontrastes, die eine erleichterte Bandenzuordnung und - durch Auflösung komplexer Banden in ihre Einzelkomponenten - eine noch gezieltere Analyse der einzelnen Spektralbereiche erlaubt. Obwohl auch alle genannten Verfahrensvarianten vorteilhaft im Wege einer direkten automatischen Datenverarbeitung durchgeführt werden können, hat sich für eine automatische Auswertung der Spektren insbesondere bei großen Probenzahlen eine vorhergehende Datenreduktion durch Methoden der multivariaten Datenanalyse, wie z.B. Korrespondenzanalyse, als zweckmäßig erwiesen, die sich auch bei Identifizierungsaufgaben mit Hilfe der FT-IR-Spektroskopie bewährt haben (Naumann, D., Fijala, V., Labischinski, H., Mikrochim. Acta [Wien] I, 373-377 (1988)). Besonders vorteilhaft erweist sich darüber hinaus, daß das neue Verfahren wegen der auf heutigen Spektrometern in der Regel digital anfallenden Spektrendaten leicht so gestaltet werden kann, daß eine automatische, rechnergesteuerte Verfahrensdurchführung und Auswertung möglich wird, so daß unter Einschluß von ebenfalls heute automatisiert durchführbaren und in der Empfindlichkeitsprüfung und Mikrobiologie allgemein eingesetzten Komponenten, wie Beimpfungseinheiten, eine automatische Apparatur zur Verfahrensdurchführung zusammengestellt werden kann. Eine Rechnersteuerung des Verfahrens ist auch deshalb von größerem Interesse, weil damit eine einfache Möglichkeit besteht, in der in konventionellen Empfindlichkeitstests üblichen Weise, den Grad der Empfindlichkeit in wenigen Skalenstufen (z.B. resistent, mäßig empfindlich, sensitiv) durch Vergleich der erhaltenen Daten mit vorher registrierten und auf dem Rechnersystem elektronisch gespeicherten Referenzdaten von Mikroorganismen mit bereits bekannter Empfindlichkeit zu definieren, ohne daß der Benutzer der Apparatur selbst (z.B. klinischer Mikrobiologe) typische Arbeitsschritte eines Spektroskopikers durchführen muß.

Fig. 1 zeigt ein Grobschema des Verfahrensablaufs (Prinzip),

Fig. 2 zeigt die zweite Ableitung eines FT-IR-Spektrums intakter Zellen von S. aureus SG 511,

Fig. 3 zeigt die mittlere Änderung der Peakhöhen P (willkürliche Einheiten) in den ersten 30 Minuten nach Zugabe des Antibiotikums.

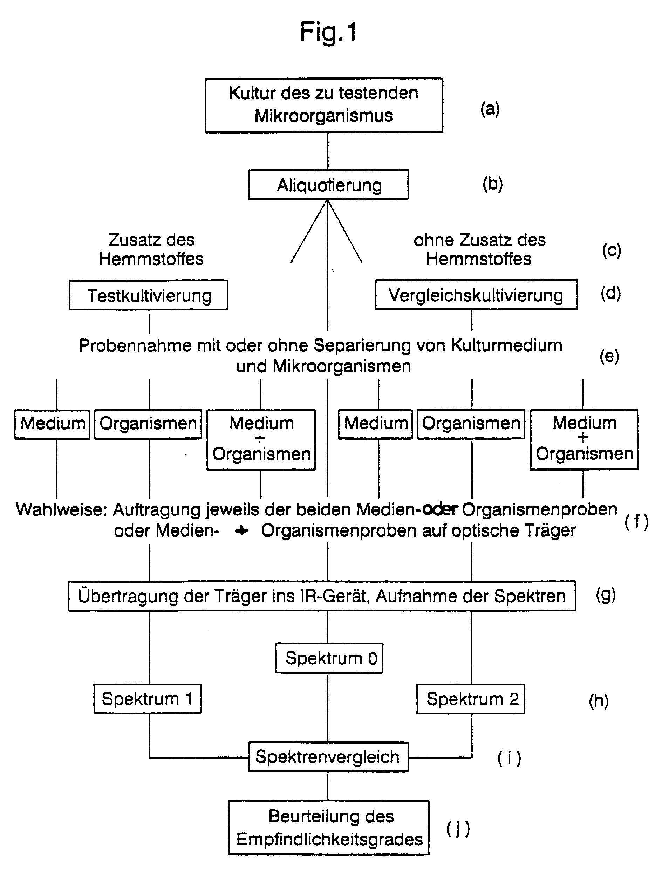

Die typischen Einzelschritte von bevorzugten Ausführungsformen des Verfahrens sind in Fig. 1 schematisch zusammengefaßt. Insgesamt erlaubt das neue Verfahren zur Prüfung der Wirksamkeit von Agenzien, wie Chemotherapeutika und/oder Antibiotika auf das Wachstum von Mikroorganismen in der geschilderten Weise Aussagen über die Wirksamkeit des Agenz bzw. Hemmstoffes je nach gewählter Verfahrensvariante bereits nach weniger als eine Stunde nach Einsatz der Reinkultur.

Auf folgende Weise wurde z.B. eine Probe von Staphyococcus aureus SG 511 auf seine Empfindlichkeit gegenüber Chloramphenicol bei einer Konzentration von 20 µg/ml des Hemmstoffes getestet:

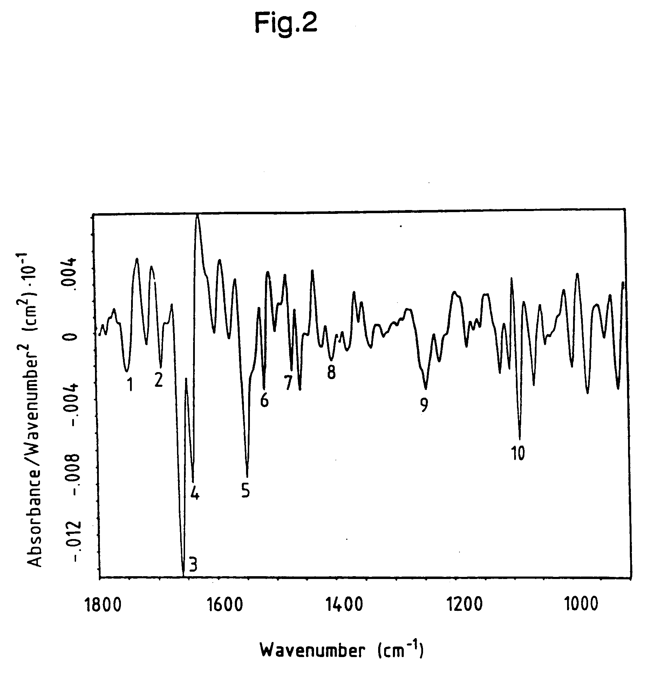

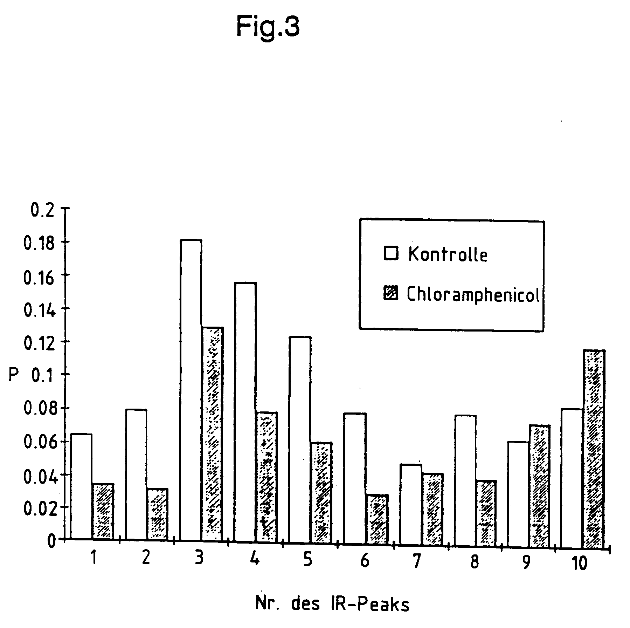

Von einer Flüssigkultur des Mikroorganismus in Peptonwasser (2,5% Bacto-Pepton, Fa. Difco, 0,5% NaCl, pH 7,0, Wachstum bei 37°C unter Schütteln) wurde 2 x 1 ml bei einer optischen Dichte der Kultur von 0,5 (gemessen bei 578 nm) für die Wirksamkeitsprüfung entnommen. Eine Probe wurde mit dem Hemmstoff (20 µg/ml) versetzt, die Kontrollprobe nicht. Nach 30 Minuten weiterer Kultivierungsdauer wurden die Zellen aus beiden Proben durch Zentrifugation in einer Laborzentrifuge (10.000 U/Minute, 4 Minuten) vom Kulturmedium getrennt. Die Sedimente wurden anschließend jeweils 1 Mal mit eiskalter 0,9% NaCl-Lösung und 2 Mal mit jeweils 1 ml eiskaltem Wasser gewaschen und in jeweils 50 µl eiskaltem Wasser resuspendiert. Jeweils 25 µl wurden auf zwei Probenfenster einer Küvette mit ZnSe-Fenstern aufgetragen. Bei leichtem Unterdruck (ca. 50 mbar) wurden die Proben in einem Exsikkator über $P_4O_{10}$ innerhalb von 2-3 Minuten zu transparenten Filmen angetrocknet und zur Vermessung in ein FT-IR-Gerät überführt. Die Proben- und Kontrollspektren wurden mit Hilfe der Transmissionstechnik auf einem FT-IR-Spektrometer MX 1-E der Fa. Nicolet ohne zusätzliche Strahlfokussierung aufgenommen. Drei Minuten Scan-Zeit (Auflösung 4 cm$^{-1}$) genügten zur Registrierung von Spektren mit gutem Signal-Rausch-Verhältnis. Bereits die visuelle Inspektion der Spektren der Proben ohne und mit Wachstum in Gegenwart des Hemmstoffes zeigte eine Empfindlichkeit des Keims gegen Chloramphenicol in der angewandten Konzentration, da insbesondere die Amid I- und II-Absorption der Probe, die in Gegenwart des Hemmstoffes kultiviert wurde, gegenüber der Kontrollprobe signifikant zurückgeblieben waren. Für eine detaillierte Auswertung wurden die Spektrendaten über eine RS-232 Schnittstelle auf einen externen Prozeßrechner transferiert. Zur Vertiefung der Aussagekraft auch in Hinsicht auf den Wirkmechanismus des Agenz werden vorzugsweise die zweiten Ableitungen der Spektren berechnet. Die Fig. 2 zeigt die so erhaltene 2. Ableitung des Spektrums der Kontrollprobe. Einige zur Auswertung herangezogene spezifische Absorptionsbanden Nr. 1 - 10 sind in der gleichen Abbildung ebenfalls gekennzeichnet. Die eigentliche Wirksamkeitsprüfung wurde in diesem Beispiel besonders detailliert durch den Vergleich der mittleren Änderung der Peakhöhen (in Absorbance-Einheiten pro Minute Kultivierungsdauer) für die in Fig. 2 dargestellten 10 ausgewählten Absorptionsbanden innerhalb der ersten 30 Minuten nach Zugabe des Hemmstoffes durchgeführt. Das Ergebnis dieses Vergleiches, für das ein weiteres Spektrum der Mikroorganismen vor Zugabe des Hemmstoffes als Bezugspunkt diente, ist als Fig. 3 für die in Fig. 2 genannten Absorptionsbanden dargestellt. Auf Grund des um bis zu 50 % geringeren Anstiegs der Absorption aller in die Auswertung einbezogenen Amid-Bandenkomponenten bzw. direkt auf den Proteingehalt ansprechenden Absorptionsbandenkomponenten (Fig.2, Bandenkomponenten Nr, 2, 3, 4, 5, 6) ergibt sich direkt die Wirksamkeit des verwendeten Antibiotikums in der gewählten Konzentration gegenüber den getesteten Mikroorganismen. Gleichzeitig zeigt die Analyse der Bandenkomponenten 9 und 10 (vgl. Fig. 2), daß die Zellwandsynthese des Mikroorganismus, wie bei Proteinsynthesehemmern zu erwarten, trotz Anwesenheit des Hemmstoffes weiterläuft bzw. sogar leicht gesteigert ist. Die weiterlaufende Zellwandsynthese ist auch einer der Gründe dafür, daß eine Wirksamkeitsprüfung nach herkömmlichen Methoden über z.B. Trübungsmessungen in diesem Zeitintervall (30 Minuten Kultivierung in Anwesenheit des Hemmstoffes) noch keine Aussage über die Wirksamkeit des Hemmstoffes ermöglicht, da die optische Dichte der Kulturen mit und ohne Hemmstoff in diesem Zeitraum sich nicht unterscheidet.

Beispiele und Bemerkungen zu Fig. 1

(a) z.B. klinisches Isolat
(b) z.B. auf feste Kulturplatten oder in flüssige Nährmedien
(c) z.B. Antibiotika, Chemotherapeutika
(d) z.B. für 30 Minuten.
Als zusätzliche Referenz kann ein Spektrum der Probe vor Zugabe des Hemmstoffes genutzt werden.
(e) z.B. Abnehmen der Kultur mit Platinöse von der Agaroberfläche oder Abzentrifugieren der Organismen vom Kulturmedium
(f) z.B. ZnSe, $BaF_2$-Scheiben für Transmissionsmessungen, oder für Messungen in ATR-Technik, reflektierende Träger (z.B. polierter Stahl) für Reflektionsmessungen etc., wahlweise angetrocknete oder flüssige Proben
(g) z.B. FT-IR-Gerät im mittleren Infrarotbereich
(h)
0 =    Referenzspektrum vor Zugabe des Hemmstoffes, kann ggf. entfallen
1 =    Spektrum nach Hemmstoff-Einwirkung
2 =    Spektrum ohne Hemmstoff-Einwirkung
(i) z.B. durch Auswertung der integralen Absorption in ausgewählten Spektralbereichen, Analyse von Peakhöhenveränderungen, Berechnung von Korrelationskoeffizienten, multivariate Datenanalyse, o.ä.
(j) direkt oder ggf. nach Standardisierung im Vergleich mit Referenzdaten

Bemerkungen zu den Fig. 2 und 3

1: >C=O-Ester-Streckschwingung bei 1740 $cm^{-1}$
2,3,4: Unterschiedliche Amid I-Komponenten der Proteine bzw. der Peptide bei 1690, 1660 und 1635 $cm^{-1}$
5: Amid II-Komponente bei 1550 $cm^{-1}$
6: "Tyrosin"-Peak bei 1510 $cm^{-1}$
7: >$CH_2$-Gruppen der Fettsäuren bei 1470 $cm^{-1}$
8: $COO^-$, symmetrische Streckschwingung bei 1400 $cm^{-1}$
9: >$PO_2^-$,asymmetrische Streckschwingung bei 1245 $cm^{-1}$
10: Intensivste Komponente der Zuckerringschwingung der Polysaccharide bei 1085 $cm^{-1}$

## Patentansprüche

1. Verfahren zur schnellen Prüfung der Wirksamkeit von Hemmstoffen oder Wachstumsstoffen auf Mikroorganismen, wie Bakterien und Pilze, dadurch gekennzeichnet, daß die Wirksamkeitsbestimmung bereits in einem sehr frühen Einwirkungsstadium des Hemmstoffes oder Wachstumsstoffes auf dem Mikroorganismus durchgeführt wird, indem die Mikroorganismen auf den in der mikrobiologischen Empfindlichkeitsprüfung allgemein verwandten Nährmedien entweder in flüssiger oder fester Form bei für die jeweiligen Mikroorganismen geeigneten Wachstumstemperaturen zwischen 15 und 45°C unter Einwirkung des Agenz während kurzer Zeitdauer kultiviert werden, daß danach von den kultivierten Mikroorganismen und/oder deren Kulturmedium Infrarotspektren aufgenommen werden und die Wirksamkeitsprüfung des jeweils verwandten Agenz durch den Vergleich der Infrarotspektren mit Infrarotspektren von ohne Zusatz des Agenz aber bei ansonsten exakt analoger Einstellung aller Kulturbedingungen, wie Nährmedienart, Zeitdauer, Temperatur, pH-Wert, kultivierten gleichen Mikroorganismen und/oder durch Vergleich mit einem vor der Kultivierung unter ansonsten gleichen Bedingungen registrierten Spektrum vorgenommen wird.

2. Verfahren nach Anspruch 1, dadurch gekennzeichnet, daß zur Wirksamkeitsprüfung ein Vergleich der integralen Intensitäten der gemessenen Absorptionsbanden in einem oder mehreren beliebigen Wellenzahlintervallen des Infrarotspektrums mit bzw. ohne Behandlung mit dem zu prüfenden Agenz vorgenommen wird.

3. Verfahren nach Anspruch 1, dadurch gekennzeichnet, daß die Wirksamkeitsprüfung über die Analyse der Veränderungen einzelner ausgewählter Absorptionsbanden oder auch mehrerer, definierter Banden nach Bandenzuordnung vorgenommen wird.

4. Verfahren nach einem der vorhergehenden Ansprüche, dadurch gekennzeichnet, daß die Mikroorganismen während einer Zeitdauer von weniger als 5 Stunden, vorzugsweise weniger als 4 Stunden, insbesondere während einer Zeitdauer zwischen 5 Minuten und 2 Stunden kultiviert werden.

5. Verfahren nach einem der vorhergehenden Ansprüche, dadurch gekennzeichnet, daß gleichzeitig mehrere mit einem Agenz versetzte Mikroorganismen in getrennten Ansätzen kultiviert und spektroskopiert werden, wobei verschiedene Mengen und/oder verschiedene Arten von Agenzien eingesetzt werden.

6. Verfahren nach einem der vorhergehenden Ansprüche, dadurch gekennzeichnet, daß zur Wirksamkeitsprüfung qualitative Veränderungen im Infrarotspektrum festgestellt und ausgewertet werden.

7. Verfahren nach einem der vorhergehenden Ansprüche, insbesondere nach Anspruch 6, dadurch gekennzeichnet, daß zur Wirksamkeitsprüfung quantitative Veränderungen im Infrarotspektrum festgestellt und ausgewertet werden.

8. Verfahren nach einem der vorhergehenden Ansprüche, dadurch gekennzeichnet, daß die zu prüfenden Kulturen direkt ohne weitere Trennnung von Medien und/oder anderen Bestandteilen infrarotspektroskopisch vermessen werden.

9. Verfahren nach einem der vorhergehenden Ansprüche, insbesondere nach Anspruch 8, dadurch gekennzeichnet, daß für die Beurteilung der Empfindlichkeit der Mikroorganismen gegenüber den zu testenden Agenzien ein Vergleich mit bereits vorher vermessenen Referenzdateien von bekannt empfindlichen und resistenten Mikroorganismen durchgeführt wird, insbesondere indem die Einstufung des Empfindlichkeitsgrades durch automatischen, zweckmäßigerweise computergesteuerten Vergleich der aktuellen spektralen Kenngrößen mit den gespeicherten Kenngrößen der Referenzdatei vorgenommen wird.

10. Verfahren nach einem der vorhergehenden Ansprüche, dadurch gekennzeichnet, daß die Wirksamkeitsprüfung mittels mathematischer Methoden der Differenz- und Derivativspektroskopie, gegebenenfalls unter Zuhilfenahme mathematischer Techniken, insbesondere Spektrenglättung, Spektrendekonvolution, Spektrenfilterung, Datenreduktion durch Korrespondenzanalyse und/oder Methoden der multivariaten Statistik etc. durchgeführt wird.

11. Verfahren nach einem der vorhergehenden Ansprüche, dadurch gekennzeichnet, daß man mit Hilfe der dispersiven Infrarotspektroskopie und-/oder der Fourier Transform-Infrarot-Spektroskopie, bzw. mit optischen Filtern arbeitet.

12. Verfahren nach einem der vorhergehenden Ansprüche, dadurch gekennzeichnet, daß die Mikroorganismen oder Kulturmedien mit Hilfe der Transmissions-, der "äußeren" Reflexions-, der "inneren" Reflexions(ATR)-, der diffusen Reflexions- und/oder der photoakustischen Spektroskopie und/oder eines IR-Mikroskops auf den üblichen transmittierenden bzw. reflektierenden Trägermaterialien, wie z.B. AgCl, AgBr, ZnSe, Ge und KRS-5 etc., Silberfolien, Alufolien und/oder polierten Stahlplättchen etc. spektroskopiert werden.

13. Verfahren nach einem der vorhergehenden Ansprüche, dadurch gekennzeichnet, daß die Mikroorganismenproben als wäßrige Suspension und/oder als Abstriche und/ oder als angetrocknete Filme und/oder als gefriergetrocknete Substanzen direkt oder in irgendeiner der an sich bekannten Matrixsubstanzen vermessen werden.

14. Verwendung des Verfahrens nach einem der vorhergehenden Ansprüche zur Messung des Grades der Wirksamkeit des Agenz.

15. Verwendung des Verfahrens nach einem der vorhergehenden Ansprüche zur Aufklärung des Wirkungsmechanismus des Agenz.

## Claims

1. Method for rapid testing of the effectivenes of inhibitors or growth substances on microorganisms, such as bacteria and fungi, characterized in that the effectiveness determination is already carried out at a very early stage of the action of the inhibitor or growth substance on the microorganism, in that the microorganisms are cultured for a brief period on the nutrient media generally used in microbiological sensitivity testing, either in liquid or solid form, at growth temperatures of between 15 and 45°C suitable for the particular microorganisms, under the action of the agent, and in that infrared spectra of the cultured microorganisms and/or their culture medium are then recorded and the effectiveness test on the particular agent used is carried out by comparison of the infrared spectra with infrared spectra of the same microorganisms cultured without the addition of the agent but with otherwise exactly analogous setting of all culture conditions, such as type of nutrient medium, time duration, temperature and pH value, and/or by comparison with a spectrum recorded before the culture under otherwise identical conditions.

2. Method as claimed in claim 1, characterized in that, for the effectiveness test, a comparison of the integral intensities of the absorption bands measured in one or more arbitrary wavenumber ranges of the infrared spectrum with and without treatment with the agent to be tested is carried out.

3. Method as claimed in claim 1, characterized in that the effectiveness test is carried out by means of analysis of the changes in individual selected absorption bands or in several defined bands in accordance with band characterization.

4. Method as claimed in any of the preceding claims, characterized in that the microorganisms are cultured for a period of less than 5 hours and preferably of less than 4 hours, in particular for a period of between 5 minutes and 2 hours.

5. Method according to one of the preceding claims, characterized in that several microorganisms to which an agent has been added are cultured, and measured spectroscopically, in separate batches at the same time, whereby different amounts and/or different types of agents are employed.

6. Method according to one of the preceding claims, characterized in that, for the effectiveness test, qualitative changes in the infrared spectrum are determined and evaluated.

7. Method according to one of the preceding claims, in particular according to claim 6, characterized in that, for the effectiveness test, quantitative changes in the infrared spectrum are determined and evaluated.

8. Method according to one of the preceding claims, characterized in that the cultures to be tested are measured directly by infrared spectroscopy, without further separation of media and/or their constituents.

9. Method according to one of the preceding claims, in particular according to claim 8, characterized in that, for assessment of the sensitivity of the microorganisms to the agents being tested, a comparison is carried out with previously measured reference databases of micoorganisms known to be sensitive and resistant, in particular by grading the degree of sensitivity by means of automated, appropriately computer-controlled, comparison of the actual spectral characteristics with the stored characteristics in the reference database.

10. Method according to one of the preceding claims, characterized in that the effectiveness test is carried out by means of mathematical methods of difference and derivative spectroscopy, if appropriate with the aid of mathematical techniques, in particular spectrum smoothing, spectrum deconvolution, spectrum filtering, data reduction by means of correspondence analysis and/or multivariance statistical methods etc.

11. Method according to one of the preceding claims, characterized in that the procedure is carried out with the aid of despersive infrared spectroscopy and/or Fourier transform infrared spectroscopy or using optical filters.

12. Method according to one of the preceding claims, characterized in that the microorganisms or culture media are subjected to spectroscopic measurement with the assistance of transmission spectroscopy, "outer" reflection spectroscopy, "inner" reflection (ATR) spectroscopy, diffuse reflection spectroscopy and/or photoacoustic spectroscopy and/or of an IR microscope on the customary transmitting or reflecting carrier materials, such as, for example, AgCl, AgBr, ZnSe, Ge and KRS-5 etc., silver foils, aluminium foils and/or polished steel platelets, etc.

13. Method according to one of the preceding claims, characterized in that the microorganism samples are measured in the form of an aqueous suspension and/or in the form of smears and/or in the form of dried films and/or in the form of freeze-dried substances, directly or in any of the matrix substances which are, in and of themselves, known in the art.

14. Use of the method as claimed in any of the preceding claims for measuring the degree of effectiveness of the agent.

15. Use of the method as claimed in any of the preceding claims to clarify the mechanism of action of the agent.

## Revendications

1. Procédé pour tester rapidement l'efficacité d'inhibiteurs ou de substances de croissance sur des micro-organismes, tels que des bactéries et des champignons, caractérisé en ce que la détermination d'efficacité est déjà réalisée à un stade très antérieur de l'action de l'inhibiteur ou de la substance de croissance sur le micro-organisme, en ce que les micro-organismes sont cultivés pendant une brève période de temps sur les milieux nutritifs généralement utilisés dans les tests de sensibilité microbiologique, soit sous la forme liquide, soit sous la forme solide, à des températures de croissance comprises entre 15 et 45°C, convenant pour les micro-organismes particuliers, sous l'action de l'agent, et en ce que les spectres infrarouges des micro-organismes cultivés et/ou de leur milieu de culture sont ensuite enregistrés, et le test d'efficacité sur l'agent particulier employé est mis en oeuvre par comparaison des spectres infrarouges, avec les spectres infrarouges des mêmes micro-organismes cultivés sans l'addition de l'agent mais avec à part cela un réglage exactement analogue de toutes les conditions de culture, telles que le type de milieu nutritif, la durée, la température et le pH et/ou par comparaison avec un spectre enregistré avant la culture sous des conditions à part cela identiques.

2. Procédé suivant la revendication 2, caractérisé en ce que, pour le test d'efficacité, on réalise une comparaison des intensités intégrales des bandes d'absorption mesurées dans un ou plusieurs intervalles de chiffres d'onde arbitraires du spectre infrarouge avec et sans traitement avec l'agent à tester.

3. Procédé suivant la revendication 1, caractérisé en ce que le test d'efficacité est réalisé grâce à une analyse des changements se produisant dans les bandes d'absorption choisies individuelles ou dans plusieurs bandes définies, suivant la caractérisation de ces bandes.

4. Procédé suivant l'une quelconque des revendications précédentes, caractérisé en ce que les micro-organismes sont cultivés pendant une période de moins de 5 heures, et de préférence de moins de 4 heures, en particulier pendant une période comprise entre 5 minutes et 2 heures.

5. Procédé suivant l'une quelconque des revendications précédentes, caractérisé en ce que plusieurs micro-organismes auxquels un agent a été ajouté sont cultivés, et soumis à des mesures au spectroscope, en lots séparés en même temps, de manière à utiliser des quantités différentes et/ou des types différents d'agents.

6. Procédé suivant l'une quelconque des revendications précédentes, caractérisé en ce que, pour le test d'efficacité, les changements qualitatifs dans le spectre infrarouge sont déterminés et évalués.

7. Procédé suivant l'une quelconque des revendications précédentes, en particulier suivant la revendication 6, caractérisé en ce que, pour le test d'efficacité, les changements quantitatifs dans le spectre infrarouge sont déterminés et évalués.

8. Procédé suivant l'une quelconque des revendications précédentes, caractérisé en ce que les cultures à tester sont mesurées directement par une spectroscopie dans l'infrarouge, sans autre séparation des milieux et/ou de leurs constituants.

9. Procédé suivant l'une quelconque des revendications précédentes, en particulier suivant la revendication 8, caractérisé en ce que, pour la vérification de la sensibilité des micro-organismes aux agents à tester, une comparaison est réalisée avec des bases de données de référence mesurées antérieurement pour les micro-organismes connus comme étant sensibles et résistants, en particulier en classant le degré de sensibilité grâce à une comparaison automatisée, contrôlée de façon appropriée par ordinateur, des caractéristiques spectrales effectives, avec les caractéristiques mémorisées dans les bases de données de référence.

10. Procédé suivant l'une quelconque des revendications précédentes, caractérisé en ce que le test d'efficacité est réalisé grâce à des méthodes mathématiques de spectroscopie différentielle et dérivative, à l'aide de techniques mathématiques, si cela convient, en particulier une uniformisation du spectre, une déconvolution du spectre, une filtration du spectre, une réduction de données grâce à une analyse de correspondance, et/ou des méthodes statistiques à multi-variance, etc.

11. Procédé suivant l'une quelconque des revendications précédentes, caractérisé en ce que le processus est mis en oeuvre à l'aide d'une spectroscopie infrarouge dispersive et/ou d'une spectroscopie infrarouge à transformée de Fourier, ou en utilisant des filtres optiques.

12. Procédé suivant l'une quelconque des revendications précédentes, caractérisé en ce que les micro-organismes ou les milieux de culture sont soumis à une mesure spectroscopique à l'aide d'une spectroscopie de transmission, d'une spectroscopie par réflexion "extérieure", d'une spectroscopie par réflexion "intérieure" (ATR), d'une spectroscopie par réflexion diffuse et/ou d'une spectroscopie photo-acoustique et/ou à l'aide d'un microscope à infrarouge sur les matériaux de support traditionnels de transmission ou de réflexion, tels que, par exemple, AgCl, AgBr, ZnSe, Ge et KRS-5, etc., des feuilles d'argent, des feuilles d'aluminium et/ou des plaquettes en aciers polis, etc.

13. Procédé suivant l'une quelconque des revendications précédentes, caractérisé en ce que les échantillons de micro-organisme sont mesurés sous la forme d'une suspension aqueuse et/ou sous la forme de frottis et/ou sous la forme de pellicules séchées et/ou sous la forme de substances lyophilisées, et ce directement ou dans l'une quelconque des substances formant matrices qui sont connues en soi en pratique.

14. Utilisation du procédé suivant l'une quelconque des revendications précédentes pour la mesure du degré d'efficacité de l'agent.

15. Utilisation du procédé suivant l'une quelconque des revendications précédentes, pour clarifier le mécanisme d'action de l'agent.

# Fig.1

```
                    ┌─────────────────────────┐
                    │  Kultur des zu testenden │        (a)
                    │     Mikroorganismus      │
                    └─────────────────────────┘
                                 │
                          ┌──────────────┐
                          │ Aliquotierung│              (b)
                          └──────────────┘
                              ╱   │   ╲
```

Zusatz des                              ohne Zusatz des        (c)
Hemmstoffes                             Hemmstoffes

┌────────────────────┐                  ┌──────────────────────┐
│  Testkultivierung  │                  │  Vergleichskultivierung │   (d)
└────────────────────┘                  └──────────────────────┘

Probennahme mit oder ohne Separierung von Kulturmedium          (e)
                 und Mikroorganismen

┌────────┐┌───────────┐┌──────────┐  ┌────────┐┌───────────┐┌──────────┐
│ Medium ││ Organismen││  Medium  │  │ Medium ││ Organismen││  Medium  │
└────────┘└───────────┘│    +     │  └────────┘└───────────┘│    +     │
                       │Organismen│                         │Organismen│
                       └──────────┘                         └──────────┘

Wahlweise: Auftragung jeweils der beiden Medien-**oder** Organismenproben
   oder Medien-  **+**  Organismenproben auf optische Träger        ( f )

┌──────────────────────────────────────────────────────────────┐
│ Übertragung der Träger ins IR-Gerät, Aufnahme der Spektren     │   (g)
└──────────────────────────────────────────────────────────────┘

                       ┌────────────┐
                       │ Spektrum 0 │
                       └────────────┘

┌────────────┐                        ┌────────────┐
│ Spektrum 1 │                        │ Spektrum 2 │              (h)
└────────────┘                        └────────────┘

               ┌──────────────────┐
               │ Spektrenvergleich│                               ( i )
               └──────────────────┘

            ┌────────────────────┐
            │   Beurteilung des  │
            │ Empfindlichkeitsgrades │    ( j )
            └────────────────────┘

# Fig.2

# Fig.3

Nr. des IR-Peaks